# FASCICULE DE BREVET EUROPEEN

(11) **EP 0 440 572 B1**
(45) Date de publication et mention de la délivrance du brevet: **26.10.1994**
(21) Numéro de dépôt: 91440006.4
(22) Date de dépôt: 01.02.1991
(51) Int. Cl.: A61F 11/08, H04R 25/00

(54) **Dispositif de transmission du son à filtrage sélectif, destiné à être mis en place dans le conduit auditif externe et son procédé de fabrication**
Im äusseren Gehörgang einsetzbares Schallübertragungsgerät mit selektivem Sieben, sowie Verfahren zur dessen Herstellung
Sound transmission device with selective filtering to be placed in the external ear canal and process of manufacturing thereof

(30) Priorité: 01.02.1990 FR 9001319
(43) Date de publication de la demande: 07.08.1991
(73) Titulaire: Le Her, Francois, F-76520 Boos (FR)
(72) Inventeur: Le Her, Francois, F-76520 Boos (FR)

(56) Documents cités:
- EP-A- 0 112 594
- EP-A- 0 197 630
- DE-U- 8 713 595
- FR-A- 2 108 657
- FR-A- 2 631 815
- US-A- 2 619 960
- US-A- 2 785 675
- US-A- 2 888 921
- US-A- 4 372 904

## Description

La présente invention a pour objet un dispositif de transmission du son à filtrage sélectif destiné à être mis en place dans le conduit auditif externe ainsi que son procédé de fabrication.

On connaît à ce jour divers dispositifs destinés à la protection individuelle contre le bruit, parmi lesquels les casques anti-bruit et les bouchons d'oreille.

Les casques anti-bruit présentent généralement une bonne efficacité au plan de la protection acoustique, toutefois ils posent le problème de leur tolérance mécanique et acoustique, du fait que leur efficacité est directement proportionnelle à la pression exercée sur le contour de l'oreille, et du fait également du phénomène d'autophonie qu'ils induisent.

Les bouchons d'oreille peuvent consister en des boules de cire molle que l'on pétrit avant de les introduire dans le conduit auditif externe, mais qui présentent l'inconvénient d'être difficiles à mettre en place correctement, donnant lieu à un contact aléatoire avec le conduit auditif externe, et l'inconvénient supplémentaire de ne pas écarter le risque d'une contamination microbienne.

Afin de remédier à cet inconvénient on a proposé un bouchon protecteur moulé directement sur le conduit auditif. Un tel bouchon fait l'objet du brevet français 2,108,657, qui en préconise le moulage dans le conduit auditif en état d'extension, de manière à assurer un ajustage plus étroit dudit bouchon dans ledit conduit auditif.

Toutefois ce type de bouchon, s'il offre effectivement l'avantage d'assurer un contact précis et anatomique avec le conduit auditif externe, présente en contrepartie l'inconvénient de ne pas éviter le phénomène d'autophonie et d'être d'une hygiène incertaine, du fait que le produit siliconé injecté dans le conduit auditif se polymérise avec l'ensemble des sérosités qui s'y trouvent présentes.

D'autre part, afin de réduire le phénomène d'autophonie lié à l'utilisation de ce type de bouchon, diverses tentatives ont été effectuées, visant à réaliser des bouchons protecteurs munis d'un perçage. Cependant la longueur et le diamètre de ces perçages, ainsi que le volume résiduel existant entre le tympan et l'extrémité du bouchon, conditionnent l'atténuation acoustique, et leur caractère aléatoire entraîne des modifications considérables de la réponse acoustique obtenue.

Pour remédier à cet inconvénient on a purposé, dans le document EP-A-0 112 594, sur lequel le préambule de la revendication 1 se fonde des valves acoustiques dont le perçage est quantifié en diamètre et en longueur, avec ou sans élément absorbant. Toutefois le filtre acoustique ainsi réalise est un filtre de deuxième ordre dont la pente d'atténuation est de seulement 15 décibels par octave.

La présente invention a pour but de remédier à ces inconvénients des systèmes connus en proposant un dispositif protecteur de l'ouïe réalisé sur mesure d'après un moulage individuel et adapté au contexte acoustique du porteur.

La présente invention a ainsi pour objet un dispositif de transmission du son à filtrage sélectif un embout selon la revendication 1.

Le dispositif composte obturant complètement le conduit auditif externe et une valve acoustique insérée dans cet embout, ainsi qu'un tube ouvert associé à au moins une cavité de résonance, selon le principe connu du résonateur HELMHOLTZ, le filtre acoustique ainsi constitué étant un filtre de quatrième ordre dont la pente d'atténuation est de 30 décibels par octave.

L'embout du dispositif selon l'invention est réalisé par moulage à partir d'une empreinte prise sur le porteur, selon les techniques utilisées en prothèse auditive.

La réalisation de cet embout doit néanmoins répondre à certaines conditions dont le respect conditionne la qualité du produit et donc du dispositif.

Ainsi, après avoir été taillée et préparée, l'empreinte est baignée dans un bain de cire chaude afin d'obtenir une surcharge qui peut être parfaitement quantifiée. Le contre-moule est ensuite réalisé en une ou deux parties puis la matière choisie est coulée dans le moule et cuite sous pression afin de réaliser sa parfaite polymérisation.

L'embout est ensuite démoulé et enduit d'un vernis, les deux opérations de vernissage consécutives permettant d'obtenir une surépaisseur de l'embout par rapport à l'empreinte, ce qui a pour effet de provoquer, lors de sa mise en place dans le conduit auditif externe, une légère compression des parois de ce dernier, empêchant toute fuite acoustique.

La matière choisie pour réaliser l'embout du dispositif selon l'invention est de préférence un silicone médical anallergique, mais elle peut aussi être un polyméthylméthacrylate polymérisé à haute pression et haute température.

La valve acoustique est mise en place dans l'embout une fois démoulé après perçage d'une ouverture tubulaire et d'un logement adapté à la recevoir.

Conformément à l'invention, la valve acoustique insérée dans l'embout comprend au moins une cavité de résonance prolongée par un tube qui est placé dans un perçage tubulaire ménagé dans l'embout et qui débouche dans une cavité résiduelle existant entre l'embout et le tympan, chaque cavité de résonance pouvant être vide ou remplie d'un élément acoustiquement absorbant comme une mousse synthétique ou de l'ouate de cellulose, selon le degré de l'absorption sonore recherché.

Selon la fréquence des vibrations sonores à absorber, le diamètre du perçage du tube ouvert peut varier de 0,1 à 2,2 mm et sa longueur de 10 à 25 mm, tandis que chaque cavité de résonance présente un volume variant de 0,1 à 3 cm³.

Dans le cas où la valve acoustique comprend plus d'une cavité de résonance, ces cavités peuvent être soit extérieures à l'embout, soit insérées totalement ou partiellement dans l'embout.

Selon une variante de réalisation, destinée à permettre au porteur de communiquer avec un correspondant dans une ambiance sonore, la valve acoustique comporte, dans une de ses cavités de résonance, un écouteur relié par un dispositif de connexion approprié à un émetteur-récepteur radio, et relié d'autre part, par un câble électrique, à un micro logé dans une cavité ménagée dans l'embout, à courte distance de la cavité résiduelle, à laquelle elle est reliée par un tube disposé dans un perçage qui joue le rôle de capteur de sons vis-à-vis des ondes sonores qui franchissent le tympan lorsque le porteur parle.

Le porteur de ce type de dispositif peut ainsi adresser, sans avoir à élever la voix, un message qui n'est pas parasité par le bruit ambiant, et recevoir un message en retour.

Selon une autre variante de réalisation, le micro est remplacé par un autre transducteur qui fait à la fois office de micro et d'écouteur. Dans ce cas, l'écouteur est supprimé, le transducteur étant directement relié au dispositif de connection lui-même relié à l'émetteur-récepteur radio.

La présente invention sera mieux comprise à la lecture de la description qui suit, faite en regard du dessin annexé qui en représente divers modes de réalisation pris à titre d'exemple, étant bien entendu que cette description ne présente aucun caractère limitatif vis-à-vis de l'invention.

Dans le dessin annexé :
- la figure 1 représente une vue en coupe schématique d'un premier dispositif selon l'invention.
- la figure 2 représente une vue en coupe schématique d'un second dispositif selon l'invention.
- la figure 3 représente une vue en coupe schématique d'un dispositif selon l'invention adapté à transmettre la voix du porteur à un système de radio communication à haute fréquence.

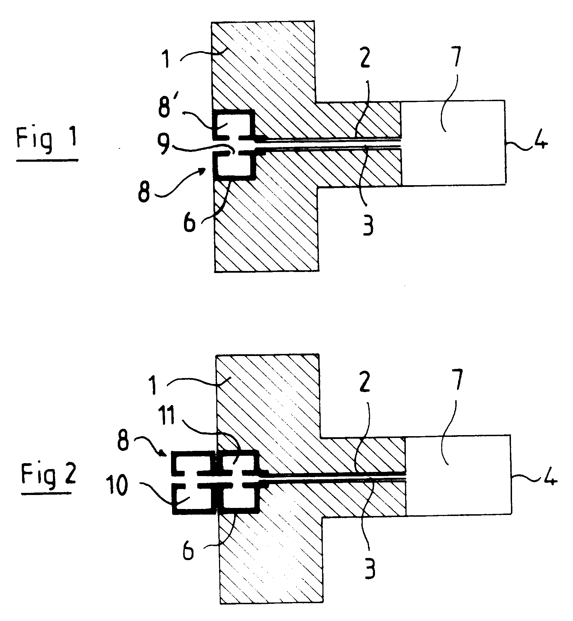

Si on se réfère d'abord à la figure 1, on voit que le dispositif selon l'invention comporte un embout 1 moulé muni d'un perçage 2 dans lequel se trouve placé un tube 3 prolongé à son extrémité externe par une valve 8 insérée dans un logement 6 ménagé dans l'embout 1 et comportant une cavité de résonance 8' munie d'une ouverture 9. Le tube 3 débouche à son extrémité interne dans une cavité résiduelle 7 ménagée entre l'extrémité interne de l'embout 1 et le tympan 4.

Il est aisé de concevoir que ce mode de réalisation permet la réalisation de toute une gamme de filtres acoustiques du 4ème ordre, en jouant sur le diamètre du tube 3, le volume de la cavité de résonance 8' de la valve 8 et le volume de la cavité résiduelle 7 existant entre l'extrémité de l'embout 1 et le tympan 4. Un tel filtre permet d'obtenir une pente de l'atténuation acoustique de 30 dB par octave.

Si on se réfère maintenant à la figure 2, le dispositif représenté sur cette figure constitue un filtre plus absorbant que le précédent, du fait que la valve 8 comprend deux cavités de résonance 10 et 11 dont la première est extérieure à l'embout 1, tandis que la seconde y est insérée, étant prolongée par un tube 3 logé dans le perçage 2 ménagé dans l'embout 1. Les différents paramètres de ce dispositif, à savoir la longueur du tube 3, le volume de chacune des cavités de résonance 10 et 11 de la valve acoustique 8 et le volume de la cavité résiduelle 7 existant entre l'embout 1 et le tympan 4 permettent également, dans ce cas de figure, de moduler à la demande le filtre acoustique ainsi constitué en l'adaptant au contexte sonore du porteur.

Selon une variante du dispositif selon l'invention, les deux valves acoustiques représentées aux figures 1 et 2 peuvent être protégées par une couronne de métal encastrable ou enclipsable dans l'embout 1 afin d'accentuer, dans certains cas particuliers, l'atténuation des basses fréquences.

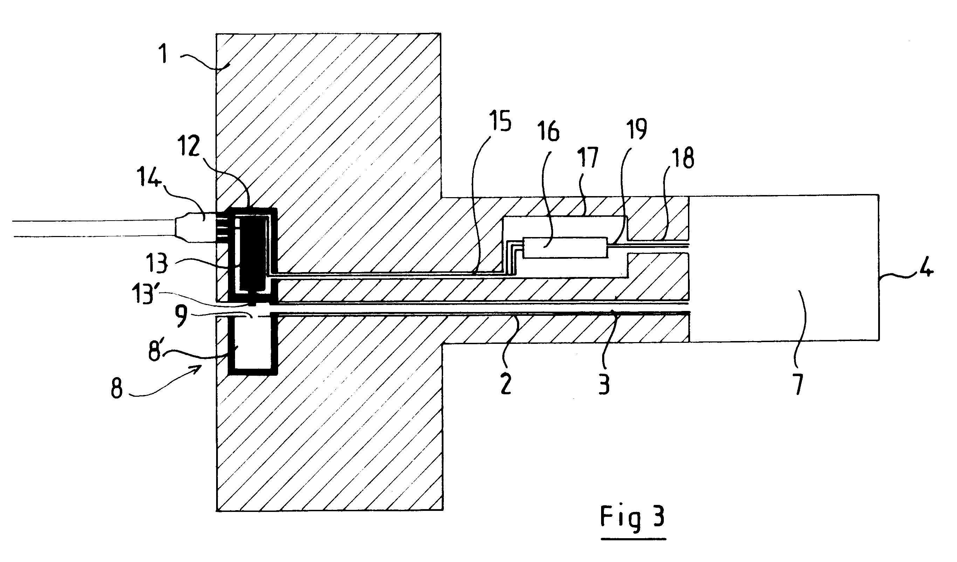

Si on se réfère à la figure 3, on voit sur cette figure un dispositif adapté à transmettre la voix du porteur à un émetteur-récepteur radio, portatif ou non. Le dispositif comporte encore dans ce cas un embout 1 muni d'un perçage 2 dans lequel est placé un tube 3 prolongé à son extrémité externe par une valve acoustique 8 délimitant une cavité de résonance 8′ dans laquelle est aménagé un logement 12 pour un écouteur 13 dont la sortie 13′ débouche dans le tube 3 et qui est relié à un dispositif de connexion 14 relié à l'émetteur-récepteur et relié d'autre part, par un câble électrique 15, à un micro 16 logé dans une cavité 17 prolongée par un perçage 18 qui débouche dans la cavité résiduelle 7 et dans lequel se trouve placé un tube 19 solidarisé au micro 16 et qui joue le rôle de capteur de sons vis-à-vis des ondes sonores qui franchissent le tympan 4 lorsque le porteur parle.

Un micro 16 de sensibilité suffisante et de courbe de réponse adaptée permet ainsi de capter dans la cavité 7 les ondes sonores de la voix du porteur sans capter les ondes sonores extérieures qui arrivent par le tuyau 3. Ce type de dispositif est particulièrement utile dans le cas où le porteur et son correspondant évoluent dans un milieu sonore particulièrement bruyant, du type de ceux où sont actionnées en permanence certaines machines-outils, leur permettant de communiquer sans que le micro du poste émetteur capte les bruits extérieurs.

Le micro 16 peut être remplacé par un autre transducteur qui fait à la fois office de micro et d'écouteur. Dans ce cas l'écouteur 13 est supprimé et le câble électrique 15 relie directement le connecteur 14 au transducteur 16.

Il va de soi que la présente invention ne saurait être limitée à la description qui précède de certains de ses modes de réalisation, susceptibles de subir un certain nombre de modifications sans pour autant sortir du cadre de l'invention.

## Revendications

1. Dispositif de transmission du son à filtrage sélectif, destine à être mis en place dans le conduit auditif externe et comportant un embout (1) muni d'un perçage (2) et d'une valve acoustique (8) insérée partiellement ou totalement dans un logement (6) ménagé dans ledit embout (1), lequel est réalisé par moulage du conduit auditif effectué par prise d'empreinte et fabrication d'un contre-moule, caractérisé en ce qu'il comporte, placé dans le perçage (2), un tube (3) débouchant par son extrémité interne dans la cavité résiduelle (7) existant entre l'embout (1) et le tympan (4) et par son extrémité externe dans la valve acoustique (8), laquelle délimite au moins une cavité de résonance (8', 10, 11).

2. Dispositif selon la revendication 1, caractérisé en ce que la cavité de résonance (8', 10, 11) peut être extérieure à l'embout (1) ou insérée dans l'embout (1).

3. Dispositif selon la revendication 1 ou la revendication 2, caractérisé en ce que la cavité de résonance (8', 10, 11) est remplie d'un matériau acoustiquement absorbant.

4. Dispositif selon l'une quelconque des revendications précédentes, caractérisé en ce que le tube (3) présente une longueur de 10 à 25 mm et un diamètre de 0,1 à 2,2 mm.

5. Dispositif selon l'une quelconque des revendications précédentes, caractérisé en ce que chaque cavité de résonance (8', 10, 11) présente un volume de 0,1 à 3 cm3.

6. Dispositif selon l'une quelconque des revendications précédentes, caractérisé en ce qu'il comporte une cavité supplémentaire (17) permettant d'abriter un transducteur relié par un câble électrique (15) à un dispositif de connexion (14).

7. Dispositif selon la revendication 6, caractérisé en ce que le transducteur est un micro (16) relié par un câble électrique (15) au dispositif de connexion (14) relié d'autre part à un écouteur (13) placé dans un logement (12) ménagé dans la valve acoustique (8) et dont la sortie (13') débouche dans le tube (3), la cavité (17) se prolongeant par un perçage (18) qui débouche dans la cavité résiduelle (7) et dans lequel se trouve placé un tube (19) destiné à capter les ondes sonores de la voix du porteur pour les transmettre à un système émetteur-récepteur radio relié au dispositif de connexion (14).

8. Dispositif selon l'une quelconque des revendications précédentes, caractérisé en ce que l'embout (1) est réalisé en silicone réticulant à haute température ou en un polyméthylméthacrylate dont la polymérisation complète est obtenue par cuisson sous pression avant démoulage.

9. Dispositif selon l'une quelconque des revendications précédentes, caractérisé en ce que l'embout (1) est recouvert d'une couche d'un vernis silicone.

10. Procédé de fabrication du dispositif selon l'une des revendications 1 à 9, par moulage à partir d'une empreinte, caractérisé en ce que préalablement à la réalisation du contre-moule on immerge l'empreinte dans un bain de cire chaude de manière à réaliser une surcharge qui peut être parfaitement quantifiée, que l'on réalise ensuite le moule à partir de l'empreinte ainsi surchargée, que l'on coule dans le moule le matériau choisi que l'on cuit sous pression, que l'on démoule l'embout et qu'on l'enduit ensuite d'un vernis en procédant à deux opérations de vernissage consécutives.

## Claims

1. Device for the transmission of selectively filtered sound intended to be placed in the external auditory canal and comprising an ear piece (1) provided with a boring (2) and an acoustic valve (8) partially or totally inserted in a housing (6) provided in the said ear piece (1) which is formed from a moulding of the auditory canal effected by the taking of a imprint and the maring of a complimentary mould, characterised in that it comprises a tube (3) placed in the boring (2), its internal end opening into the residual cavity (7) existing between the ear piece (1) and the eardrum (4) and its outer end opening into the acoustic valve (8) which defines at least a resonant cavity (8', 10, 11).

2. A device according to claim 1 characterised in that the resonant cavity (8', 10, 11) may be outside the ear piece (1) or inserted in the ear piece (1).

3. A device according to claim 1 or claim 2 characterised in that the resonant cavity (8', 10, 11) is filled with a acoustically absorbent material.

4. A device according to any one of the preceding claims characterised in that the tube (3) has a length of from 10-25mm and a diameter of from 0.1-2.2mm.

5. A device according to any one of the preceding claim characterised in that each resonant cavity (8', 10, 11) has a volume of from 0.1-3cm³.

6. A device according to any one of the preceding claims characterised in that it comprises a supplementary cavity (17) allowing the shielding of a transducer connected by a electric cable (15) to a connection device (14).

7. A device according to claim 6 characterised in that the transducer is a microphone (16) connected by a electric cable (15) to a connection device (14) connected furthermore to a speaker (13) placed in a housing (12) provided in the acoustic valve (8) and whose exit (13') opens into the tube (3), the cavity (17) extending by means of a boring (18) which opens into the residual cavity (7) and in which is placed a tube (19) intended to capture the sound waves of the voice of the wearer in order to transmit them to a radio emitter/receiver system connected to the connection device (14).

8. A device according to any one of the preceding claims characterised in that the ear piece (1) is formed of a silicone cross linking at high temperature or from a polymethylmethacrylate whose complete polymerisation is obtained by curing under pressure before turning out from the mould.

9. A device according to any one of the preceding claims characterised in that the ear piece (1) is covered with a layer of a silicone varnish.

10. Procedure for the fabrication of a device according to any of claims 1-9 by moulding from an imprint characterised in that prior to the formation of the complimentary mould, the imprint is immersed in a bath of hot wax in such a way as to produce an overcoat which may be exactly quantified, then the mould is obtained from the thus overcoated imprint, the chosen material is then flowed into the mould which is cured under pressure, the block is then turned out from the mould and coated with a varnish in the process of two consecutive varnishing operations.

## Patentansprüche

1. Vorrichtung zur selektiv-gefilterten Schallübertragung zum Einsetzen in den äußeren Gehörgang mit einem Stopfen (1), der eine Öffnung (2) und ein akustisches Ventil (8) aufweist, welches sich teilweise oder vollständig in einer Aufnahme (6) des Stopfens (1) befindet, wobei der Stopfen dem Gehörgang durch Abnehmen eines Abdrucks und Herstellung einer Negativform angepaßt ist, **dadurch gekennzeichnet**, daß sie innerhalb der Öffnung (2) eine Röhre (3) aufweist, deren inneres Ende zu dem zwischen dem Stopfen (1) und dem Trommelfell (4) bestehenenden Resthohlraum (7) hin geöffnet ist und deren äußeres Ende zu dem akustischen Ventil (8) hin geöffnet ist, welches mindestens einen Resonanzhohlraum (8', 10, 11) aufweist.

2. Vorrichtung nach Anspruch 1, **dadurch gekennzeichnet**, daß der Resonanzhohlraum (8', 10, 11) sich außerhalb des Stopfens (1) befindet oder in dem Stopfen (1) versenkt ist.

3. Vorrichtung nach einem der Ansprüche 1 oder 2, **dadurch gekennzeichnet**, daß der Resonanzhohlraum (8', 10, 11) mit einem akustisch absorbierenden Material gefüllt ist.

4. Vorrichtung nach einem der voranstehenden Ansprüche, **dadurch gekennzeichnet**, daß die Röhre (3) eine Länge zwischen 10 und 25 mm und einen Durchmesser zwischen 0,1 und 2,2 mm aufweist.

5. Vorrichtung nach einem der voranstehenden Ansprüche, **dadurch gekennzeichnet**, daß jeder Resonanzhohlraum (8', 10, 11) ein Volumen zwischen 0,1 und 3 cm³ aufweist.

6. Vorrichtung nach einem der voranstehenden Ansprüche, **dadurch gekennzeichnet**, daß sie einen zusätzlichen Hohlraum (17) aufweist, zur Aufnahme eines Meßwandlers, der über ein elektrisches Kabel (15) mit einer Anschlußvorrichtung (14) verbunden ist.

7. Vorrichtung nach Anspruch 6, **dadurch gekennzeichnet**, daß der Meßwandler ein Mikrophon (16) ist, welches über ein elektrisches Kabel (15) mit der Anschlußvorrichtung (14) verbunden ist, die auf der anderen Seite mit einem Hörer (13) innerhalb einer Aufnahme (12) des akustischen Ventils (8) verbunden ist, dessen Ausgang (13') in die Röhre (3) mündet, wobei der Zusatzhohlraum (17) über eine Bohrung (18) mit dem Resthohlraum (7) verbunden ist innerhalb derer sich eine Röhle (19) befindet, wodurch die Schallwellen der Stimme des Trägers aufgenommen und über ein an die Anschlußvorrichtung (14) angeschlossenes Radio-Sender-Empfänger-System übertragen werden können.

8. Vorrichtung nach einem der voranstehenden Ansprüche, **dadurch gekennzeichnet**, daß der Stopfen (1) aus einem bei hoher Temperatur vernetzten Silikon besteht oder aus einem Polymethylmetacrylat dessen vollständige Polymerisation durch Erhitzen unter Druck vor der Entfernung aus der Form bewirkt wird.

9. Vorrichtung nach einem der voranstehenden Ansprüche, **dadurch gekennzeichnet**, daß der Stoffen (1) mit einer Firnisschicht aus Silikon bedeckt ist.

10. Verfahren zur Herstellung einer Vorrichtung nach einem der Ansprüche 1 bis 9 durch Formung aus einem Abdruck, **dadurch gekennzeichnet**, daß vor der Herstellung der Negativform der Abdruck in ein heißes Wachsbad getaucht wird, um eine perfekt zu quantifizierende Überladung zu erhalten, daraufhin die Form ausgehend von dem überladenen Abdruck hergestellt wird, in die Form das gewählte Material eingegossen und unter Druck erhitzt wird, der Stopfen aus der Form entfernt und eine Firnisschicht in zwei aufeinanderfolgenden Verfahrensschritten aufgebracht wird.
